# EUROPÄISCHE PATENTSCHRIFT

(11) **EP 1 541 540 B1**
(45) Veröffentlichungstag und Bekanntmachung des Hinweises auf die Patenterteilung: **02.04.2008**
(21) Anmeldenummer: 04027034.0
(22) Anmeldetag: 13.11.2004
(51) Int. Cl.: C07C 29/70, C07C 31/30

(54) **Verfahren zur Herstellung alkoxidreiner Magnesiumalkoxide**
Process for the preparation of pure magnesium alkoxides
Procédé pour la préparation d' alcoolates de magnesium purs

(30) Priorität: 13.12.2003 DE 10358412
(43) Veröffentlichungstag der Anmeldung: 15.06.2005
(73) Patentinhaber: Evonik Degussa GmbH, 40474 Düsseldorf (DE)
(72) Erfinder: Rauleder, Hartwig, Dr., 79618 Rheinfelden (DE); Standke, Burkhard, Dr., 79540 Lörrach (DE)

(56) Entgegenhaltungen:
- EP-A- 0 026 547
- GB-A- 667 708
- GB-A- 767 601

## Beschreibung

Die Erfindung betrifft ein Verfahren zur Herstellung von Magnesiumalkoxiden durch Alkoholyse. Das Verfahren basiert auf dem Austausch von Alkoxidgruppen eines Magnesiumalkoxids in Gegenwart eines anderen Alkohols als dem zum ursprünglichen Alkoxid korrespondierenden Alkohol, d. h. der Alkoholyse eines Magnesiumalkoxids mit einem anderen Alkohol.

Magnesiumdialkoxide, nachfolgend auch kurz Erdalkalialkoxide genannt, werden in vielfältiger Weise in der organisch synthetischen Chemie eingesetzt.

Das gebräuchlichste Verfahren zu ihrer Herstellung ist die direkte Umsetzung von Erdalkalimetall mit Alkohol unter Wasserstoffabspaltung [Liebigs Annalen der Chemie 444, 236 (1925)]. Diese Umsetzung ist bei Verwendung längerkettiger Alkohole aufgrund der geringen Affinität der Reaktionspartner zueinander außerordentlich erschwert.

US 2 965 663 lehrt die Umsetzung von Metallen der Gruppen IA, IIA und IIIA des Periodensystems der Elemente (PSE) mit Alkoholen zu entsprechenden Alkoxiden nach einem speziellen Rückflußverfahren. Nachteilig sind die außerordentlich langen Reaktionszeiten insbesondere bei Einsatz von Metallen der Gruppen IIA und IIIA.

DE-OS 22 61 386 offenbart, daß man die Umsetzung von Erdalkalimetall und Alkohol bei höheren Temperaturen rascher durchführen kann, jedoch mit dem Nachteil, daß man die Umsetzung in einem Autoklaven unter hohem Druck durchführen muss.

Die GB 667,708 beschreibt ein Verfahren zur Herstellung von Magnesia, wobei man metallisches Magnesium oder eine Magnesiumlegierung mit wasserfreiem Ethyl oder Methylalkohol unter Bildung von Magnesiumalkoholat umsetzt, das Magnesiumalkoholat mit wasserunlöslichem Alkohol behandelt, so dass der Ethylalkohol oder der Methylalkohol in dem Magnesiumalkoholat durch den wasserunlöslichen Alkohol ersetzt wird, den entstandenen Ethylalkohol und Methylalkohol abtrennt, das Magnesiumalkoholat mit Wasser hydrolysiert und das entstandene Magnesia von dem wasserunlöslichen Alkohol abtrennt.

Ein generelles Problem bei der Herstellung von Erdalkalialkoxiden aus Erdalkalimetall und Alkohol ist der Restgehalt an nicht umgesetztem Metall, der bei der weiteren Verwendung des Produkts stört, zum Beispiel , wenn man das Magnesiumalkoxid als Katalysator in der organisch synthetischen Chemie einsetzt.

Eine weitere Route zur Herstellung von Metallalkoxiden, insbesondere solche höherer Alkohole, ist die Alkoholyse (Kirk-Othmer Encyclopedia of Chemical Technology, 3rd Ed., Vol. 2, Seite 8 und 9). Nachteilig bei diesem Verfahren ist, daß bei der Herstellung von Alkoxiden der Gruppe IIA des PSE eine im Produkt verbleibende Verunreinigung an Alkohol beziehungsweise Alkoxid des niedrigeren Alkohols zu beobachten ist. So verbleiben beispielsweise bei der Alkoholyse von Magnesiumethanolat mit Isopropanol zu Magnesium-iso-propylat noch ca. 15 Gew.-% Ethanolat gerechnet als Ethanol im Produkt. Auch ein solcher Gehalt an Fremdalkoxid im Produkt kann sich beispielsweise bei der Verwendung von Magnesium-di-iso-propanolat als Katalysator bei einer Synthese in der organischen Chemie als störend auswirken.

Der vorliegenden Erfindung lag somit die Aufgabe zugrunde, ein weiteres, möglichst wirtschaftliches Verfahren zur Herstellung höherer Magnesium alkoxide mit hinreichender Produktreinheit bereitzustellen.

Die Aufgabe wird erfindungsgemäß entsprechend den Merkmalen der Patentansprüche gelöst.

Überraschenderweise wurde gefunden, daß man in einfacher und gleichzeitig wirtschaftlicher Weise ein metallfreies und alkoxidreines Magnesiumalkoxid der allgemeinen Formel I

M(OR¹)₂ (I),

worin M für Magnesium steht und R¹ eine lineare, verzweigte oder cyclische Alkylgruppe mit 2 bis 20 C-Atomen, vorzugsweise mit 2 bis 10 C-Atomen, darstellt, herstellen kann, wenn man eine in Lösung befindlichen Verbindung der allgemeinen Formel II

M(OR²)ₓ(OR³)_{y}(OR⁴)_{z} (II),

worin M für Magnesium, Gruppen R², R³ und R⁴ gleich oder verschieden sind, und eine lineare Alkylgruppe mit 1 bis 4 C-Atomen darstellen und der Maßgabe 0 ≤ x ≤ 2, 0 ≤ y ≤ 2, 0 ≤ z ≤ 2 mit (x+y+z) = 2, mit einem im Überschuss eingesetzten Alkohol der allgemeinen Formel III

HOR¹ (III),

worin R¹ dieselbe Bedeutung besitzt wie in Formel I, verschieden von Gruppen R², R³ und R⁴ gemäß Formel II ist und in der Alkylkette mindestens eine C-Atom mehr besitzt als die längste Alkylgruppe aus der Reihe R², R³ und R⁴, umsetzt, dadurch gekennzeichnet daß man die Verbindungen der Formel I und/oder der Formel II während der Alkoholyse zumindest anteilig gelöst hält und wobei man den während der Umsetzung entstehenden Alkohol HOR², HOR³ und/oder HOR⁴ destillativ aus dem Reaktionsgemisch entfernt sowie einen Alkohol der allgemeinen Formel III vorlegt und ein Magnesiumalkoxid der allgemeinen Formel II unter guter Durchmischung zugibt.

Erfindungsgemäß ist beim vorliegenden Verfahren insbesondere dafür Sorge zu tragen, daß man zur Durchführung der Alkoholyse ein solches Startalkoxid, (vergleiche Verbindungen der Formel II), einsetzt und/oder bei der vorliegenden Umsetzung eine Reaktionszwischenstufe entsteht, beispielsweise ein Mischalkoxid, und/oder ein Zielalkoxid, das heißt ein Produkt der Formel I, entsteht, welches in dem für die Umsetzung verwendeten Alkohol der Formel III zumindest anteilig löslich ist.

Ferner wird das Reaktionsgemisch zur Durchführung der Alkoholyse geeigneterweise erwärmt. Die vorliegende Umsetzung wird durch die destillative Entfernung des bei der Alkoholyse frei werdenden Alkohols vervollständigt:

M(OR²)ₓ(OR³)_{y}(OR⁴)_{z} + 2 R¹OH → M(OR¹)₂ + x HOR² + y HOR³+ z HOR⁴

Beispielhaft sei die Alkoholyse von Magnesiumdimethanolat gelöst in Methanol mit n-Hexanol angeführt:

Mg (OMe)₂ + 2 HexOH → Mg(OHex)₂ + 2 MeOH

Weiterhin sind insbesondere
Mg (O-n-Propyl)₂,
Mg (O-i-Propyl)₂,
Mg(O-n-Butyl)₂, Mg(O-n-Butyl)₂
Mg(O-i-Butyl)₂, Mg(O-i-Butyl)₂
nach dem erfindungsgemäßen Verfahren zugänglich.

Unter metallfreien Magnesiumalkoxiden versteht man bei der vorliegenden Erfindung solche, die weniger als 0,04 Gew.-% Magnesiummetall, bezogen auf das Magnesiumalkoxid enthalten. Bei der vorliegenden Erfindung erhält man bevorzugt metallfreie Magnesiumalkoxide der allgemeinen Formel I mit ≤ 0,03 Gew.-% Magnesiummetall, insbesondere solche mit ≤ 0,02 Gew.-% Magnesiummetall bis hin zur Nachweisgrenze des Magnesiummetalls, wobei die Angabe jeweils auf das Magnesiumalkoxid bezogen ist.

Unter alkoxidreinen Magnesiumalkoxiden versteht man bei der vorliegenden Erfindung solche, die < 10 Gew.-% Fremdalkoxid, gerechnet als Alkohol und bezogen auf das gewünschte Magnesiumalkoxid, enthalten. Bei der vorliegenden Erfindung erhält man bevorzugt alkoxidreine Magnesiumalkoxide der allgemeinen Formel I mit ≤ 5,0 Gew.-% Fremdalkoxid, besonders vorzugsweise solche mit ≤ 1,0 Gew.-% Fremdalkoxid bis hin zur Nachweisgrenze des betreffenden Fremdalkoxids oder Alkohols unter den gegeben Bedingungen, wobei die Angabe jeweils auf das Magnesiumalkoxid bezogen ist.

Gegenstand der vorliegenden Erfindung ist daher ein Verfahren zur Herstellung von einem metallfreien und alkoxidreinen Magnesiumalkoxid der allgemeinen Formel I

M(OR¹)₂ (I),

worin M für Magnesium steht und R¹ eine lineare, verzweigte oder cyclische Alkylgruppe mit 2 bis 20 C-Atomen darstellt,
durch Alkoholyse einer Verbindung der allgemeinen Formel II

M(OR²)ₓ(OR³)_{y}(OR⁴)_{z} (II),

worin M für Magnesium steht, Gruppen R², R³ und R⁴ gleich oder verschieden sind, und eine Alkylgruppe mit 1 bis 4 C-Atomen darstellen und der Maßgabe . 0 ≤ x ≤ 2, 0 ≤ y ≤ 2, 0 ≤ z ≤ 2 mit (x + y + z) = 2,
mit einem im Überschuss eingesetzten Alkohol der allgemeinen Formel III

HOR¹ (III),

worin R¹ dieselbe Bedeutung besitzt wie in Formel I, verschieden von Gruppen R², R³ und R⁴ gemäß Formel II ist und in der Alkylkette mindestens eine C-Atom mehr besitzt als die längste Alkylgruppe aus der Reihe R², R³ und R⁴, dadurch gekennzeichet daß man die Verbindungen der Formel I und/oder der Formel II während der Umsetzung zumindest anteilig gelöst hält, und man den während der Umsetzung entstehenden Alkohol HOR², HOR³ und/oder HOR⁴ destillativ aus dem Reaktionsgemisch entfernt sowie einen Alkohol der allgemeinen Formel III vorlegt und ein Magnesiumalkoxid der allgemeinen Formel II unter guter Durchmischung zugibt.

Beim erfindungsgemäßen Verfahren setzt man vorzugsweise Magnesiumdimethanolat oder Magnesiumdiethanolat ein.

Weiterhin wird beim erfindungsgemäßen Verfahren als Verbindung der allgemeinen Formel III Ethanol, n-Propanol, i-Propanol, n-Butanol, sek-Butanol, t-Butanol, n-Pentanol, Amylalkohol, n-Hexanol, n-Octanol, i-Octanol oder n-Decanol bevorzugt eingesetzt.

Zur Durchführung des erfindungsgemäßen Verfahrens legt man vorzugsweise einen Alkohol der allgemeinen Formel III vor und gibt ein Magnesiumalkoxid der allgemeinen Formel II unter guter Durchmischung zu. Geeigneterweise kann man das Magnesiumalkoxid in Pulverform oder in dispergierter Form oder in gelöster Form zugeben. Insbesondere ist bevorzugt, daß man das Magnesiumalkoxid in Methanol und/oder Ethanol gelöst oder in Methanol und/oder Ethanol dispergiert zugibt. Bevorzugt werden homogene Lösungen des Magnesiumalkoxids eingesetzt.

Um die erfindungsgemäße Alkoholyse besonders rasch durchführen zu können, kann man bei einer höheren Temperatur als die Umgebungstemperatur arbeiten. Vorzugsweise führt man die erfindungsgemäße Umsetzung bei einer Temperatur im Bereich von 20 °C bis hin zu den Siedepunkten der jeweils vorliegenden Alkohole unter Normaldruck. Insbesondere arbeitet man bei einer Temperatur im Bereich von 90 bis 140 °C. Geeigneterweise entfernt man bei der Durchführung der erfindungsgemäßen Alkoholyse den während der Umsetzung entstehenden Alkohol HOR², HOR³ und/oder HOR⁴ destillativ aus dem Reaktionsgemisch; hierzu kann man auch Vakuum anlegen. Man sollte bei der Durchführung des vorliegenden Verfahrens ferner darauf achten, dass das verwendete Destillationssystem über eine hinreichende Trennleistung verfügt. Vorzugsweise arbeitet man zur destillativen Entfernung des genannten Alkohols aus dem Reaktionsgemischs bei einem Druck von kleiner 0,1 bis 1,1 bar abs.

Die erfindungsgemäße Umsetzung führt man bevorzugt solange unter Normaldruck oder gegebenenfalls unter leicht verringertem Druck durch, bis am Kopf der Kolonne die entsprechende Siedetemperatur des Alkohols mit dem höchsten Siedepunkt für mindestens eine Stunde festzustellen ist.

Magnesiummethanolat, geeigneterweise hergestellt durch Lösen von Magnesium in Methanol, ist im Allgemeinen mit einer Konzentration von bis zu 10 Gew.-% in Methanol löslich und wird daher beim erfindungsgemäßen Verfahren als Edukt, d. h. als Startalkoholat der allgemeinen Formel II bevorzugt. Ebenfalls bevorzugt man für eine vorteilhaften

Insbesondere kann man Alkoxide der Formel II erfindungsgemäß mit höheren Alkoholen, wie zum Beispiel n-Hexanol oder n-Octanol umsetzen.

Im Allgemeinen führt man das erfindungsgemäße Verfahren wie folgt aus:

Zunächst stellt man ein Magnesiumalkoxid der allgemeinen Formel II M(OR²)ₓ(OR³)_{y}(OR⁴)_{z} in an sich bekannter Weise her. Dazu kann man Magnesiummetall M in einem zum jeweiligen Alkoxid korrespondierenden Alkohol beziehungsweise Alkoholgemisch, bestehend aus HOR², HOR³ und/oder HOR⁴, vorzugsweise Methanol oder Ethanol, umsetzen. Man kann die Oberfläche des eingesetzten Magnesium Metalls zusätzlich vorreinigen, um ein besseres Anspringen der Reaktion zu erreichen. Ferner kann man einen Katalysator zusetzen, beispielsweise Jod. Trennt man nach der Umsetzung den überschüssigen Alkohol ab, kann man ein pulverförmiges Magnesiumalkoxid erhalten. Üblicherweise enthält ein so hergestelltes Magnesiumalkoxid einen Rest Magnesiummetallanteil von ≥ 0,04 Gew.-%, bezogen auf das Magnesiumalkoxid insbesondere dann, wenn Alkohole mit mehr als 2 C-Atomen verwendet werden. In der Regel handhabt man das Magnesiumalkoxid unter Ausschluss von Feuchtigkeit und unter Schutzgasatmosphäre. Das Magnesiumalkoxid, das heißt das Edukt kann nun als alkoholische Lösung oder in Pulverform oder in dispergierter Form, beispielsweise als alkoholische Dispersion, für die erfindungsgemäße Alkoholyse eingesetzt werden. Geeigneterweise legt man den für die Alkoholyse vorgesehenen, geeigneterweise getrockneten Alkohol der allgemeinen Formel III (HOR¹) im Überschuss in einem trockenen, kühlbaren beziehungsweise beheizbaren Reaktionsgefäß mit Rührvorrichtung unter Schutzgas, beispielsweise trockenem Stickstoff oder Argon, vor und gibt das Edukt gemäß Formel II M(OR²)ₓ(OR³)_{y}(OR⁴)_{z} - dispergiert, teilgelöst oder gelöst in HOR¹, HOR², HOR³ und/oder HOR⁴ - zu, und das Edukt reagiert erfindungsgemäß mit dem Alkohol HOR¹ vorzugsweise unter Bildung von HOR², HOR³ sowie HOR⁴ und M(OR¹)₂, wobei man das Reaktionsgemisch geeigneterweise gut durchmischt, die Temperatur vorzugsweise im Bereich von 20 bis 140 °C, bevorzugt 90-140 °C, hält und während der erfindungsgemäßen Alkoholyse gleichzeitig HOR², HOR³, HOR⁴ beziehungsweise HOR¹ oder entsprechende Gemische daraus über die Gasphase, das heißt destillativ aus dem System entfernt. In der Regel erhält man so das gewünschte Produkt M(OR¹)₂ gelöst, teilgelöst oder dispergiert in dem zum Zielalkoxid korrespondierenden Alkohol (HOR¹). Nun kann man den verbliebenen Alkohol oder das verbliebene Alkoholgemisch durch Destillation oder durch Filtration vom erhaltenen Magnesiumalkoxid M(OR¹)₂ trennen.

In der Regel benötigt man zur erfindungsgemäßen Durchführung eines Ansatzes in vorteilhafter Weise weniger als 10 Stunden.

Nach dem erfindungsgemäßen Verfahren sind metallfreie und alkoxyreine Magnesiumalkoxide höherer Alkohole in einfacher und wirtschaftlicher Weise - auch in einem technischen Maßstab - zugänglich.

Die vorliegende Erfindung wird durch die nachfolgenden Beispiele näher erläutert, ohne den Gegenstand der Erfindung zu beschränken:

### Beispiele

In den nachfolgenden Beispielen 1 bis 7 liegt das Eduktalkoxid in homogener Lösung vor. Man erhält nach erfindungsgemäßer Alkoholyse Produkte mit einem Fremdalkoxidgehalt von ≤ 1 Gew.-%, gerechnet als Alkohol.

In den Beispielen 8 und 9 liegen sowohl das Produkt- als auch das Eduktalkoxid in homogener Lösung vor. Nach Alkoholyse erhält man ein Produkt mit einem Fremdalkoxidgehalt von ≤ 0,1 Gew.-%, gerechnet als Alkohol.

Sind die Ausgangs-Magnesiumalkoxide in dem verwendeten Lösemittel, das heißt im jeweiligen Alkohol oder Alkoholgemisch, schwer löslich, siehe Vergleichsbeispiel A, so erfolgt die Alkoholyse zum Zielalkoxid nur unvollständig, sodaß ein Fremdalkoxidgehalt > 10 Gew.-% im Produkt verbleibt.

### Beispiel 1

### Herstellung von Magnesium-di-iso-propanolat aus Magnesiumdimethanolat

In einer Apparatur - bestehend aus 2-l-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung - werden 1,2 kg iso-Propanol vorgelegt und zum Sieden erhitzt. Mit einer Zulaufgeschwindigkeit von ca. 15 ml/Minute wird methanolische Magnesiumdimethanolatlösung (9,5 Gew.-% Magnesiummethylat) eindosiert. Mit Beginn der Dosierung wird mit Rücklaufverhältnis 1 und 82 °C auf 65 °C fallender Kopftemperatur zunächst iso-Propanol/Methanol-Gemisch und bei 65 °C reines Methanol abdestilliert. Anschließend wird bei steigender Kopftemperatur (von 65 auf 82 °C) mit von 5 auf 20 steigendem Rücklaufverhältnis destilliert. Ist die Kopftemperatur bei 82 °C längere Zeit konstant (ca. 1 Stunde), besteht das Destillat aus iso-Propanol, und die Reaktion ist beendet. Die Gesamtdauer der Umsetzung beträgt ca. 7 Stunden. Magnesium-di-iso-propanolat ist in Propanol schwer löslich. Es liegt am Ende der Reaktion dispergiert in Propanol vor. Die Dispersion wird am Rotavapor eingedampft und bei ca. 120 °C und einem Druck von weniger als 1 mbar 3 Stunden getrocknet. Man erhält ein weißes, feinkörniges Pulver (Primärpartikel ca. 1 µm Durchmesser, agglomeriert zu 5 bis 50 µm messenden Partikeln). Der Gehalt an Methanol des isolierten Magnesium-iso-propanolats beträgt weniger als 1 Gew.-%. Der Gehalt an freiem Magnesium liegt mit weniger als 0,02 Gew.-% unter der Nachweisgrenze der gewählten Analysenmethode.

### Beispiel 2

### Herstellung von Magnesium-di-n-butanolat aus Magnesiumdimethanolat

In einer Apparatur - bestehend aus 2-1-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung - werden 1,2 kg n-Butanol (Butan-1-ol) vorgelegt und auf 95 °C erhitzt. Mit einer Zulaufgeschwindigkeit von ca. 15 ml/Minute werden 0,8 kg methanolische Magnesiumdimethanolatlösung (7,5 Gew.-% Magnesiummethylat) eindosiert. Bei von 90 auf 117 °C steigender Blasentemperatur wird bei 65 °C Kopftemperatur zunächst reines Methanol abdestilliert. Anschließend wird bei steigender Kopftemperatur (von 65 auf 117 °C) mit einem Rücklaufverhältnis von 20 Methanol/n-Butanol-Gemisch abdestilliert. Ist die Kopftemperatur bei 117°C längere Zeit konstant (ca. 1 Stunde), besteht das Destillat aus n-Butanol, und die Reaktion ist beendet. Die Reaktionszeit beträgt ca. 7 Stunden. Magnesium-di-n-butanolat ist in n-Butanol schwer löslich. Es liegt am Ende der Reaktion dispergiert in n-Butanol vor. Die Dispersion wird am Rotavapor eingedampft und bei ca. 120 °C und einem Druck von weniger als 1 mbar 3 Stunden getrocknet. Man erhält ein weißes, grobkörniges, gelartig gebrochenes Pulver mit unregelmäßiger Partikelstruktur. Der Gehalt an Methanol im isolierten Magnesium-di-n-butanolat beträgt weniger als 0,11 Gew.-%. Der Gehalt an freiem Magnesium liegt mit weniger als 0,02 Gew.-% unter der Nachweisgrenze der gewählten Analysenmethode.

### Beispiel 3

### Herstellung von Magnesium-di-sek-butanolat aus Magnesiumdimethanolat

In einer Apparatur - bestehend aus 2-1-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung - werden 0,8 kg sek-Butanol (Butan-2-ol) vorgelegt und auf 95 °C erhitzt. Mit einer Zulaufgeschwindigkeit von ca. 15 ml/Minute werden 0,2 kg methanolische Magnesiumdimethanolatlösung (7,5 Gew.-% Magnesiummethylat) eindosiert. Bei von 90 auf 99 °C steigender Blasentemperatur wird bei 65 °C Kopftemperatur zunächst reines Methanol abdestilliert. Anschließend wird bei steigender Kopftemperatur (von 65 auf 99 °C) mit einem Rücklaufverhältnis von 20 Methanol/Butanol-Gemisch abdestilliert. Ist die Kopftemperatur bei 99 °C längere Zeit konstant (ca. 1 Stunde), besteht das Destillat aus sek-Butanol, und die Reaktion ist beendet. Die Reaktionszeit beträgt ca. _7 Stunden. Magnesium-di-sekbutanolat ist in sek-Butanol schwer löslich. Es liegt am Ende der Reaktion dispergiert in sek-Butanol vor. Die Dispersion wird am Rotavapor eingedampft und bei ca. 120 °C und einem Druck von weniger als 1 mbar 3 Stunden getrocknet. Man erhält ein weißes, grobkörniges, gelartig gebrochenes Pulver mit unregelmäßiger Partikelstruktur. Der Gehalt an Methanol des isolierten Magnesium-sek-butylats beträgt weniger als 1,0 Gew.-%. Der Gehalt an freiem Magnesium liegt mit weniger als 0,02 Gew.-% unter der Nachweisgrenze der gewählten Analysenmethode.

### Beispiel 4

### Herstellung von Magnesium-di-n-amylat aus Magnesiumdimethanolat

In einer Vakuum-Apparatur - bestehend aus 2-l-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung, Vakuumpumpe LEYBOLD (D2A) und Kühlfalle (-78 °C) - werden 1,2 kg n-Amylalkohol vorgelegt und bei 600 mbar auf 90 °C erhitzt. Mit einer Zulaufgeschwindigkeit von ca. 15 ml/Minute werden 0,8 kg methanolische Magnesiumdimethanolatlösung (7,5 Gew.-% Magnesiummethylat) eindosiert. Bei von 90 auf 120 °C steigender Blasentemperatur wird zunächst bei 54 °C Kopftemperatur reines Methanol abdestilliert. Anschließend wird bei 400 mbar und bei bis auf 104 °C steigender Kopftemperatur mit einem Rücklaufverhältnis von 10 Methanol/n-Amylalkohol-Gemisch abdestilliert. Ist die Kopftemperatur bei 104 °C längere Zeit konstant (ca. 1 Stunde), besteht das Destillat aus n-Amylalkohol, und die Reaktion ist beendet. Die Reaktionszeit beträgt ca. 5 Stunden. Magnesium-di-n-amylat ist in n-Amylalkohol schwer löslich. Es liegt am Ende der Reaktion dispergiert in n-Amylalkohol vor. Die Dispersion wird am Rotavapor eingedampft und bei ca. 120 °C und einem Druck von weniger als 1 mbar 3 Stunden getrocknet. Man erhält ein weißes, grobkörniges, gelartig gebrochenes Pulver mit unregelmäßiger Partikelstruktur. Der Gehalt an Methanol des isolierten Magnesium-di-n-amylats beträgt weniger als 1,0 Gew.-%. Der Gehalt an freiem Magnesium liegt mit weniger als 0,02 Gew.-% unter der Nachweisgrenze der gewählten Analysenmethode.

### Beispiel 5

### Herstellung von Magnesium-di-n-hexanolat aus Magnesiumdimethanolat

In einer Vakuum-Apparatur - bestehend aus 2-1-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung, Vakuumpumpe LEYBOLD (D2A) und Kühlfalle (-78 °C) - werden 0,8 kg n-Hexylalkohol (Hexan-1-ol) vorgelegt und bei 500 mbar auf 100 °C erhitzt. Mit einer Zulaufgeschwindigkeit von ca. 15 ml/Minute werden 0,6 kg methanolische Magnesiumdimethanolatlösung (7,5 Gew.-% Magnesiummethylat) eindosiert. Bei von 100 auf 120 °C steigender Blasentemperatur wird zunächst bei 46 °C Kopftemperatur reines Methanol abdestilliert. Anschließend wird bei 350 mbar und bei bis auf Raumtemperatur fallender Kopftemperatur weiter destilliert. Ist die Kopftemperatur auf Raumtemperatur gefallen, ist die Reaktion beendet. Die Reaktionszeit beträgt ca. 4 Stunden. Magnesium-di-n-hexanolat ist in n-Hexylalkohol schwer löslich. Es liegt am Ende der Reaktion dispergiert in n-Hexylalkohol vor. Die Dispersion wird am Rotavapor eingedampft und bei ca. 120 °C und einem Druck- von weniger als 1 mbar 3 Stunden getrocknet. Man erhält ein weißes, grobkörniges, gelartig gebrochenes Pulver mit unregelmäßiger Partikelstruktur. Der Gehalt an Methanol des isolierten Magnesium-n-hexylats beträgt weniger als 0,1 Gew.-%. Der Gehalt an freiem Magnesium liegt mit weniger als 0,02 Gew.-% unter der Nachweisgrenze der gewählten Analysenmethode.

### Beispiel 6

### Herstellung von Magnesium-di-n-decanolat aus Magnesiumdimethanolat

In einer Apparatur - bestehend aus 1-l-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung - werden 0,25 kg n-Decylalkohol (Decan-1-ol) vorgelegt und auf 120 °C erhitzt. Mit einer Zulaufgeschwindigkeit von ca. 25 ml/Minute werden 0,25 kg methanolische Magnesiumdimethanolatlösung (7,5 Gew.-% Magnesiummethylat) eindosiert. Bei 65 °C bis auf Raumtemperatur fallender Kopftemperatur wird Methanol abdestilliert. Ist die Kopftemperatur auf Raumtemperatur gefallen, ist die Reaktion beendet. Die Reaktionszeit beträgt ca. 3 Stunden.

Magnesium-di-n-decanolat ist in n-Decylalkohol schwer löslich. Es liegt am Ende der Reaktion dispergiert in n-Decylalkohol vor. Die Dispersion wird am Rotavapor eingedampft und bei ca. 130 °C und einem Druck von weniger als 1 mbar 3 Stunden getrocknet. Man erhält ein weißes, grobkörniges, gelartig gebrochenes Pulver mit unregelmäßiger Partikelstruktur. Der Gehalt an Methanol des isolierten Magnesium-di-n-decanolats beträgt weniger als 0,1 Gew.-%. Der Gehalt an freiem Magnesium liegt mit weniger als 0,02 Gew.-% unter der Nachweisgrenze der gewählten Analysenmethode.

### Beispiel 7

### Herstellung von Magnesiumdiethanolat aus Magnesiumdimethanolat

In einer Apparatur - bestehend aus 2-l-Mehrhalskolben mit Innenthermometer, KPG-Rührer, Tropftrichter, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung - werden 0,711 kg Ethanol vorgelegt und zum Sieden erhitzt. Mit einer Zulaufgeschwindigkeit von ca. 1,75 ml/Minute werden 700 ml methanolische Magnesiumdimethanolatlösung (8,5 Gew.-% Magnesiummethylat) eindosiert. Mit Beginn der Dosierung wird mit Rücklaufverhältnis von 30 auf 10 fallend und von 78 auf 65 °C fallender Kopftemperatur zunächst Ethanol/Methanol-Gemisch und bei 65 °C reines Methanol abdestilliert. Anschließend wird bei steigender Kopftemperatur (von 65 auf 78 °C) mit einem Rücklaufverhältnis von 10 Ethanol/Methanol-Gemisch destilliert. Ist die Kopftemperatur bei 78 °C längere Zeit konstant (ca. 1 Stunde), besteht das Destillat aus Ethanol, und die Reaktion ist beendet. Die Reaktionszeit beträgt ca. 20 Stunden. Magnesiumdiethanolat ist in Ethanol schwer löslich. Es liegt am Ende der Reaktion dispergiert in Ethanol vor. Die Dispersion wird am Rotavapor eingedampft und bei ca. 120 °C und einem Druck von weniger als 1 mbar 3 Stunden getrocknet. Man erhält ein weißes, feinkörniges Pulver (Primärpartikel ca. 1 µm Durchmesser, aggregiert zu 5 bis 20 µm messenden Agglomeraten). Der Gehalt an Methanol des isolierten Magnesiumethylats beträgt weniger als 0,7 Gew.-%. Der Gehalt an freiem Magnesium liegt mit weniger als 0,02 Gew.-% unter der Nachweisgrenze der gewählten Analysenmethode.

### Vergleichsbeispiel A

### Herstellung von Magnesium-iso-propanolat aus Magnesiumdiethanolat

In einer Apparatur - bestehend aus 2-l-Mehrhalskolben mit Innenthermometer, KPG-Rührer, Destillationskolonne (Füllkörperkolonne, Innendurchmesser 25 mm, Füllhöhe 1,2 m, Maschendrahtringe 4 * 4 mm V4A), mit Kolonnenkopf (automatisch gesteuerter Flüssigkeitsteiler, Kontaktthermometer), Heizpilz und N₂-Überdeckung - werden 229 g Magnesiumethylat und 1,5 l iso-Propanol (Propan-2-ol) vorgelegt. Bei 82 °C Sumpftemperatur wird bei 78 bis 80 °C Kopftemperatur Ethanol abdestilliert (Destillationsdauer 24 Stunden). Anschließend wird im Rotavapor iso-Propanol entfernt und das verbleibende Pulver im Vakuum (p < 1 mbar, T = 80 °C) 2 Stunden getrocknet. Das isolierte Produkt enthält trotz langer Umsetzungsdauer (24 Stunden) noch 15 Gew.-% Ethanol nach Hydrolyse.

### Vergleichsbeispiel B

### Herstellung von Magnesium-di-n-propanolat im Autoklaven

In einem 10-1-Stahlautoklaven werden 112 g Magnesiumspäne und 3 000 g Propan-1-ol vorgelegt. Bei 188 °C und einem Druck von 38 bar wird die Umsetzung in insgesamt 5 Stunden durchgeführt. Anschließend wird bei einer Temperatur von 80 °C und einem Druck von ca. 50 mbar n-Propanol destillativ entfernt. Das Alkoxid wird im Anschluss bei 80 °C und einem Druck < 1 mbar getrocknet. Das Produkt enthält metallisches Magnesium in einer Menge von 0,04 Gew.-%, bezogen auf Magnesium-di-n-propanolat.

## Patentansprüche

1. Verfahren zur Herstellung von einem metallfreien, alkoxidreinen Magnesiumalkoxid der allgemeinen Formel I
M(OR¹)₂ (I),
worin M für Magnesium steht und R¹ eine lineare, verzweigte oder cyclische Alkylgruppe mit 2 bis 20 C-Atomen darstellt,
durch Alkoholyse einer in Lösung befindlichen Verbindung der allgemeinen Formel II
M(OR²)ₓ(OR³)_{y}(OR⁴)_{z} (II),
worin M für Magnesium steht, Gruppen R², R³ und R⁴ gleich oder verschieden sind, und eine lineare Alkylgruppe mit 1 bis 4 C-Atomen darstellen und der Maßgabe 0 ≤ x ≤ 2, 0 ≤ y ≤ 2 , 0 ≤ z ≤ 2 mit (x + y + z) = 2,
mit einem im Überschuss eingesetzten Alkohol der allgemeinen Formel III
HOR¹ (III),
worin R¹ dieselbe Bedeutung besitzt wie in Formel I, verschieden von Gruppen R², R³ und R⁴ gemäß Formel II ist und in der Alkylkette mindestens eine C-Atom mehr besitzt als die längste Alkylgruppe aus der Reihe R², R³ und R⁴, **dadurch gekennzeichnet, daß** die Verbindungen der Formel I und/oder der Formel II während während der Umsetzung zumindest anteilig gelöst hält, und wobei man den während der Umsetzung entstehenden Alkohol HOR², HOR³ und/oder HOR⁴ destillativ aus dem Reaktionsgemisch entfernt, sowie einen Alkohol der allgemeinen Formel III vorlegt und ein Magnesiumalkoxid der allgemeinen Formel II unter guter Durchmischung zugibt.

2. Verfahren nach Anspruch 1, **dadurch gekennzeichnet, daß** man als Verbindung der allgemeinen Formel II Magnesiumdimethanolat einsetzt.

3. Verfahren nach einem der Ansprüche 1 bis 2, **dadurch gekennzeichnet, daß** man als Verbindung der allgemeinen Formel III Ethanol, n-Propanol, i-Propanol, n-Butanol, sek-Butanol, t-Butanol, n-Pentanol, Amylalkohol, n-Hexanol, n-Octanol, i-Octanol oder n-Decanol einsetzt.

4. Verfahren nach einem der Ansprüche 1 bis 3, **dadurch gekennzeichnet, daß** man die Umsetzung solange unter Normaldruck durchführt, bis am Kopf der Kolonne die Siedetemperatur des Alkohols mit dem höchsten Siedepunkt für mindestens eine Stunde festzustellen ist.

5. Verfahren nach Anspruch 1, **dadurch gekennzeichnet, daß** man das Magnesiumalkoxid in Pulverform oder in dispergierter Form oder in gelöster Form zugibt.

6. Verfahren nach Anspruch 5, **dadurch gekennzeichnet, daß** man das Magnesiumalkoxid in Methanol und/oder Ethanol gelöst oder in Methanol und/oder Ethanol dispergiert zugibt.

## Claims

1. Process for preparing a metal-free, alkoxide-pure magnesium alkoxide of the general formula I
M(OR¹)₂ (I)
in which M stands for magnesium and R¹ represents a linear, branched or cyclic alkyl group with 2 to 20 C atoms,
by alcoholisis of a compound which is present in solution and has the general formula II
M(OR²)ₓ(OR³)_{y}(OR⁴)_{z} (II)
in which M stands for magnesium, groups R², R³ and R⁴ are the same or different and represent a linear alkyl group with 1 to 4 C atoms, with the proviso 0 ≤ x ≤ 2, 0 ≤ y ≤ 2, 0 ≤ z ≤ 2 with (x + y + z) = 2,
with an excess of an alcohol of the general formula III
HOR¹ (III)
in which R¹ has the same significance as in formula I, is different from groups R², R³ and R⁴ according to formula II, and has in the alkyl chain at least one C atom more than the longest alkyl group from the series R², R³ and R⁴, **characterized in that** the compounds of formula I and/or of formula II are kept dissolved, at least in a proportion, during the reaction and the alcohol HOR², HOR³ and/or HOR⁴ formed during the reaction is removed from the reaction mixture by distillation, and also an alcohol of the general formula III is introduced as initial charge and a magnesium alkoxide of the general formula II is added with thorough commixing.

2. Process according to Claim 1, **characterized in that** magnesium dimethanolate is used as compound of the general formula II.

3. Process according to either Claim 1 or 2, **characterized in that** ethanol, n-propanol, i-propanol, n-butanol, sec-butanol, t-butanol, n-pentanol, amyl alcohol, n-hexanol, n-octanol, i-octanol or n-decanol is used as compound of the general formula III.

4. Process according to any one of Claims 1 to 3, **characterized in that** the reaction is performed under normal pressure until the boiling point of the alcohol having the highest boiling point can be detected at the head of the column for at least one hour.

5. Process according to Claim 1, **characterized in that** the magnesium alkoxide is added in powder form or in dispersed form or in dissolved form.

6. Process according to Claim 5, **characterized in that** the magnesium alkoxide is added dissolved or dispersed in methanol and/or ethanol.

## Revendications

1. Procédé de fabrication d'un alcoxyde de magnésium exempt de métal et pur, de formule générale I
M(OR¹)₂ (I),
dans laquelle M représente le magnésium et R¹ représente un groupement alkyle linéaire, ramifié ou cyclique ayant de 2 à 20 atomes de carbone,
par alcoolyse d'un composé de formule générale II en solution
M(OR²)ₓ(OR³)_{y}(OR⁴)_{z} (II),
dans laquelle M représente le magnésium, les groupements R², R³ et R⁴ sont identiques ou différents et représentent un groupement alkyle linéaire ayant de 1 à 4 atomes de carbone, à condition que 0 ≤ x ≤ 2, 0 ≤ y ≤ 2, 0 ≤ z ≤ 2 avec (x + y + z) = 2,
avec un alcool de formule générale III utilisé en excès
HOR¹ (III),
dans laquelle R¹ a la même signification que dans la formule I, est différent des groupements R², R³ et R⁴ de la formule II et comprend dans sa chaîne alkyle au moins un atome de carbone de plus que le groupement alkyle de la série R², R³ et R⁴, les composés de formule I et/ou de formule II étant maintenus au moins proportionnellement en solution pendant la réaction, et l'alcool HOR², HOR³ et/ou HOR⁴ obtenu pendant la réaction étant éliminé du mélange réactionnel par distillation, un alcool de formule générale III et un alcoxyde de magnésium de formule générale II étant obtenus sous agitation.

2. Procédé selon la revendication 1, **caractérisé en ce que** le diméthanolate de magnésium est utilisé comme composé de formule générale II.

3. Procédé selon l'une quelconque des revendications 1 à 2, **caractérisé en ce que** l'éthanol, le n-propanol, le i-propanol, le n-butanol, le sec-butanol, le t-butanol, le n-pentanol, l'alcool amylique, le n-hexanol, le n-octanol, le i-octanol ou le n-décanol est utilisé en tant que composé de formule générale III.

4. Procédé selon l'une quelconque des revendications 1 à 3, **caractérisé en ce que** la réaction est réalisée à pression normale jusqu'à ce qu'à la tête de la colonne, la température d'ébullition de l'alcool présentant le point d'ébullition le plus élevé soit observée pendant au moins une heure.

5. Procédé selon la revendication 1, **caractérisé en ce que** l'alcoxyde de magnésium est obtenu sous forme de poudre ou sous forme dispersée ou sous forme dissoute.

6. Procédé selon la revendication 5, **caractérisé en ce que** l'alcoxyde de magnésium est dissous dans du méthanol et/ou de l'éthanol ou dispersé dans du méthanol et/ou de l'éthanol.
